# EUROPEAN PATENT SPECIFICATION

(11) **EP 2 787 956 B1**
(45) Date of publication and mention of the grant of the patent: **03.02.2016**
(21) Application number: 12791149.3
(22) Date of filing: 19.11.2012
(51) Int. Cl.: A61K 8/34, A61K 8/368, A61K 8/46, A61K 8/73, A61K 8/81, A61Q 19/00, A61Q 17/00, A61Q 11/00, A61K 8/36, A01N 49/00, A01N 31/16, A01N 31/08, A01N 31/04, A61Q 19/10

(54) **AN ANTI BACTERIAL COMPOSITION**
ANTIMIKROBIELLE ZUSAMMENSETZUNG
COMPOSITION ANTIMICROBIENNE

(30) Priority: 09.12.2011 IN MM34752011; 26.01.2012 EP 12152561
(43) Date of publication of application: 15.10.2014
(73) Proprietor: Unilever N.V., 3013 AL Rotterdam (NL); Unilever PLC, London, Greater London EC4Y 0DY (GB)
(72) Inventor: JAYARAMAN, Sujatha, Whitefield Bangalore 560 066 (IN); APPAVOO, Shanthi, Whitefield Bangalore 560 066 (IN); BARNE, Sameer, Keshav, Whitefield Bangalore 560 066 (IN); SAJI, Maya,Treesa, Whitefield Bangalore 560 066 (IN)
(74) Representative: Corsten, Michael Allan
(86) International application number: PCT/EP2012/073005
(87) International publication number: WO 2013/083393

(56) References cited:
- EP-A1- 0 916 720
- WO-A1-2006/053458
- WO-A1-2010/046238
- WO-A1-2011/036048
- WO-A1-2011/039630
- WO-A1-2011/151171
- US-A1- 2008 253 976
- DATABASE WPI Section Ch, Week 1992 Thomson Scientific, London, GB; Class A96, AN 1992-103097 XP002682076, "Mixt. for mfr. of tooth-paste - contains additional mineral Laminaria extract, thymol and sodium benzoate as antiseptics, and polyvinyl-pyrrolidone", & SU 1 644 963 A1 30 April 1991 (1991-04-30)
- SAGOO SK ET AL: "Chitosan potentiates the antimicrobial action of sodium benzoate on spoilage yeasts", LETTERS IN APPLIED MICROBIOLOGY, WILEY-BLACKWELL PUBLISHING LTD, GB, vol. 34, no. 3, 1 March 2002 (2002-03-01), pages 168-172, XP009162064, ISSN: 0266-8254, DOI: 10.1046/J.1472-765X.2002.01067.X [retrieved on 2002-03-01]
- LINA WANG ET AL: "Synergistic Antimicrobial Activities of Natural Essential Oils with Chitosan Films", JOURNAL OF AGRICULTURAL AND FOOD CHEMISTRY, vol. 59, no. 23, 29 October 2011 (2011-10-29), pages 12411-12419, XP055051544, ISSN: 0021-8561, DOI: 10.1021/jf203165k

## Description

### Technical Field

The present invention relates to a non-therapeutic method of disinfecting a surface and to an antibacterial composition. It particularly relates to an antibacterial composition for disinfecting various substrates like external surface of the human or animal body like skin and hair, the oral cavity, and hard surfaces in households or other indoor and outdoor environs.

### Background and Prior Art

Sanitizing and disinfecting compositions comprising chlorine and nascent oxygen based bleaching agents are known. Such compositions require rather long contact time to provide efficacious antibacterial action. In practice, users, in particular children, do not spend long time in cleaning and as a result, cleaning with such compositions does not provide adequate prevention from surface or topical infection or adequate protection against diseases. The user, in spite of cleaning hands, is likely to have skin with relatively inadequate bacterial removal and may cause contamination of further animate and/or inanimate surfaces and this leads to spreading of pathogens and consequent diseases. Further, many antibacterial actives in addition to abrasives are included in oral care compositions like dentifrices but these actives generally require several minutes if not hours before effective antibacterial action is achieved.

Similarly in the area of hard surface cleaning e.g. cleaning of floors, table tops or utensils, the antibacterial actives in the compositions are in contact with the substrate for less than a few minutes after which the surface is either wiped off or rinsed with water. These short time scales of cleaning action are ineffective in providing the desired benefit since most known antibacterials commonly used in such products take several hours to provide the desired kill of microbes.

The present applicants in pursuit of solving this problem have disclosed in WO2010046238 a combination of essential oil actives, thymol and terpineol that interact synergistically to provide anti-bacterial activity in very fast times, in many cases as low as 15 seconds or lesser.

EP0916720 describes liquid dishwashing compositions which have antimicrobial properties. The composition comprises a surfactant, a hydrotrope, an unsaturated aliphatic terpene alcohol or a derivative thereof, and an amphiphilic polymer having a hydrophobic and a hydrophilic portion.

SU1644963 03 discloses a toothpaste composition having antiseptic properties containing 0.2-0.3 wt.-% of polyvinyl pyrrolidone, 1.2-1.8 wt.-% of sodium carboxymethyl cellulose, 0.2-0.S wt.-% of sodium benzoate and 0.01-0.1 wt.-% of thymol.

Many essential oils actives are relatively expensive ingredients. Additionally, essential oils are also known for their strong odour; using high amounts of these gives a strong smell to the product that is not always appreciated by the consumer.

Accordingly it remains to be desired to prepare anti-bacterial compositions having a high anti-bacterial effect, even with a low dosage of anti-bacterial essential oil actives.

The present applicants in pursuit of this objective, have, in WO11151172 disclosed a combination of two essential oil actives along with a polymer of a select class to provide synergistic antibacterial action at low concentration of the essential oil actives.

Further, in WO11151171 the present applicants have disclosed a combination of two polymers along with an essential oil active to provide synergistic antibacterial action at low concentration of the essential oil actives. It has been found that from the polymers and essential oil actives chosen, a single polymer and a single essential oil active in combination do not provide the synergistic activity. It has now been found that if only one essential oil active and one polymer is used, the enhanced effect is seen only when a hydrotope of the present invention is included.

In the paper published in the Letters in Applied Microbiology 2002, 34, 168-172 titled "Chitosan potentiates the antimicrobial action of sodium benzoate on spoilage yeasts" the synergistic activity against three yeasts has been demonstrated using a combination of sodium benzoate and chitosan glutamate. There is no indication that this combination can be useful against bacteria.

The present applicants have been working on further improving this technology and in an effort to find solutions to the problem of achieving high antibacterial efficacy at even further lower concentrations of essential oil actives, have arrived at the present invention. They have found that a combination of specific essential oil actives along with a polymer of a select class in the presence of specific hydrotropes are able to achieve the high antibacterial efficacy at even lower concentration of the actives which is not achieved by each of the ingredients singly or as binary combinations.

It is therefore an object of the invention to provide an antibacterial composition, having good anti-bacterial properties, at very low levels of essential oil actives.

### SUMMARY OF THE INVENTION

According to the first aspect of the invention there is provided an antibacterial composition comprising
(a) 0.01 to 10% by weight of an essential oil active selected from the group consisting of eugenol, thymol, geraniol, terpineol, and mixtures thereof;
(b) a polymer selected from the group consisting of polymers of vinyl alcohol, chitosan, and mixtures thereof; and
(c) a hydrotrope selected from the group consisting of sodium benzoate, sodium toluene sulphonate, sodium cumene sulphonate, sodium xylene sulphonate, sodium salicylate, sodium acetate, and mixtures thereof.

Another aspect of the present invention provides for a non-therapeutic method for providing an anti-bacterial effect to a substrate comprising the steps of:
(a) applying a composition of the first aspect to the substrate, and
(b) waiting for at least 15 seconds.

According to yet another aspect, the present invention provides a non-therapeutic use of a composition comprising 0.01 to 10% by weight of an essential oil active selected from the group consisting of eugenol, thymol, geraniol, terpineol and mixtures thereof; a polymer selected from the group consisting of chitosan, polymers of vinyl alcohol, , and mixtures thereof; and a hydrotrope selected from the group consisting of sodium benzoate, sodium toluene sulphonate, sodium cumene sulphonate, sodium xylene sulphonate, sodium salicylate, or sodium acetate and mixtures thereof; for fast reduction in bacterial count.

### DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

By an antibacterial composition as used herein, is meant to include a composition for cleaning and disinfecting topical areas e.g. skin and/or hair of mammals, especially humans. Such a composition may be generally classified as leave-on or rinse off, and includes any product applied to a human body for also improving appearance, cleansing, odor control or general aesthetics. It is more preferably a rinse off product. The composition of the present invention may be in the form of a liquid but may also be modified to include a lotion, cream, foam or gel, or toner, or applied with an implement or via a face mask, pad or patch. "Skin" as used herein is meant to include skin on the face and body (e.g., neck, chest, back, arms, underarms, hands, legs, buttocks and scalp). The composition of the invention is also of relevance to applications on any other keratinous substrates of the human body other than skin e.g. hair where products may be formulated with specific aim of providing disinfection and cleaning.

By anti-septic liquid is meant a composition, usually in transparent form which may be coloured or may be substantially colourless that is used to disinfect various animate and inanimate surfaces. The transparent form is usually in micro-emulsion format where oils are dispersed in water using surfactants where the oil droplet size is so small as to be smaller than that which causes diffraction of light thereby providing a substantially transparent appearance to the composition. The anti-septic liquid is usually used after dilution with water usually in the weight ratio of 1:1 to 1:200 preferably in a ratio of 1:10 to 1:80. The diluted solution may be transparent but is preferably turbid or hazy. This liquid is usually used to disinfect surface of human or animal body. The antiseptic liquid is also used to disinfect fabric especially where it is believed to be high in microorganisms e.g. linen in hospitals, clinics, nappies, and undergarments. The antiseptic liquid may also be used for cleaning floors and other surfaces in homes e.g. in kitchens and bathrooms and in certain public places where cleanliness and disinfection are highly sought after.

The composition of the invention comprises a selected essential oil active, a selected polymer and a selected hydrotrope. This composition is especially useful since the concentration of essential oil antibacterial actives that needs to be used is low. However, the essential oil actives may also be used at higher concentrations e.g from 0.01 to 10% by weight of the composition.

The composition preferably comprises 0.01 to 5%, more preferably 0.01 to 1%, further more preferably 0.01 to 0.5% by weight of essential oil active. The essential oil active is selected from the group consisting of thymol, terpineol eugenol, geraniol, and mixtures thereof. More preferably, the essential oil active is selected from the group consisting of thymol, terpineol, eugenol, and mixtures thereof. Further more preferably, the essential active is a mixture of thymol and terpineol. Even further more preferred is a mixture of thymol, terpineol and eugenol.

### Thymol

The structure of thymol is given below:

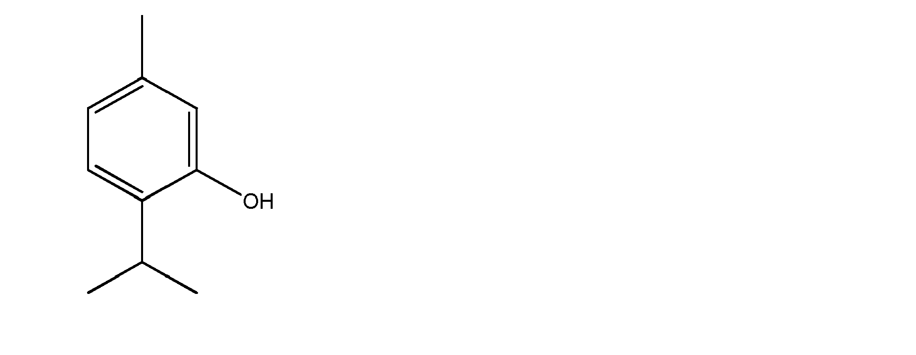

The composition of the invention comprises preferably 0.01 to 5%, more preferably 0.01 to 1%, further more preferably 0.01 to 0.4%, by weight thymol. Thymol may be added to the composition in purified form. Alternatively, thyme oil or thyme extract comprising thymol may be added to the composition, while ensuring that thymol is present in the desired concentration in the composition of the present invention. Thyme oil or thyme extract is obtained from the thyme plant. Thyme plant refers to a plant belonging be genus *Thymus* and includes but is not limited to the following species: *Thymus vulgaris, Thymus zygis, Thymus satureoides, Thymus mastichina, Thymus broussonetti, Thymus maroccanus, Thymus pallidus, Thymus algeriensis, Thymus serpyllum, Thymus pulegoide,* and *Thymus citriodorus.*

### Terpineol

The structure of a terpineol compound is given below:

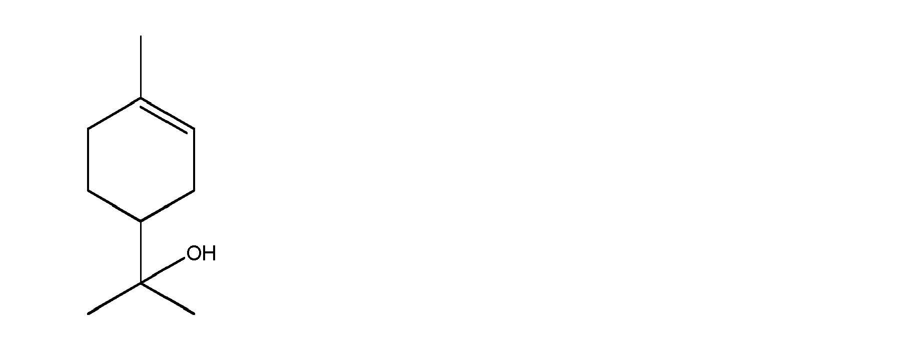

The terpineol is preferably selected from alpha-terpineol, beta-terpineol, gamma-terpineol or mixtures thereof. It is particularly preferred that the terpineol is alpha-terpineol. Terpineol may be added to the antibacterial composition in purified form. Alternatively pine oil comprising terpineol may be added to the antibacterial composition while ensuring that terpineol is present in the desired concentration in the composition of the present invention. The composition preferably comprises 0.01 to 5%, more preferably 0.02 to 5%, further more preferably 0.03 to 1%, and even more preferably 0.04 to 0.6% by weight terpineol.

### Eugenol

Eugenol is an allyl chain-substituted guaiacol. It is generally extracted from certain spices like clove or cinnamon. Eugenol has been used as a perfumery component, in preparing flavors, as an antiseptic or as a local anesthetic. The composition of the invention preferably comprises 0.005 to 5%, preferably 0.02 to 1%, more preferably 0.03 to 0.4%, by weight eugenol.

Eugenol has the structure:

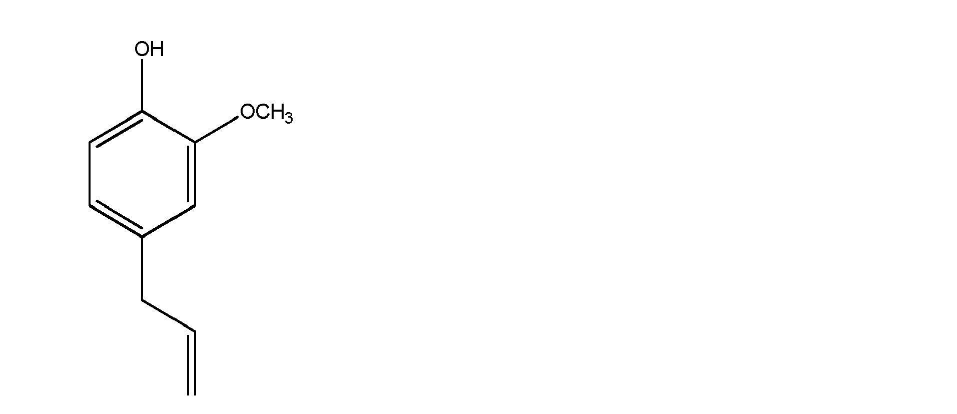

The composition may preferably comprise a combination of thymol and terpineol in any of the preferred concentrations as specified above for thymol and terpineol, respectively. For instance, the composition may preferably comprise a mixture of 0.01 to 0.6% thymol and 0.02 to 1.5% terpineol by weight of the composition. The composition of the present invention most preferably comprises a mixture of 0.01 to 0.4% eugenol, 0.01 to 0.6% thymol, and 0.02 to 1.5% terpineol by weight of the composition.

### Geraniol

The structure of the geraniol compound is given below:

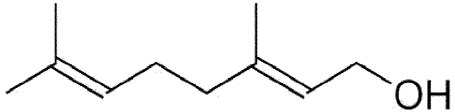

Geraniol is a monoterpenoid and an aliphatic alcohol. It is the primary component of rose oil, palmrosa oil and citronella oil. It has a rose- -like odor and is commonly used in perfumes. The composition of the invention preferably comprises 0.005 to 5%, preferably 0.02 to 1%, more preferably 0.03 to 0.4%, by weight geraniol.

The composition of the invention comprises a polymer. The polymer is selected from the group consisting of polymers of vinyl alcohol, chitosan and mixtures thereof. The polymer is preferably present in 0.001 to 25%, more preferably in 0.1 to 1.0 and most preferably in 0.1 to 0.2 % by weight of the composition.

The composition comprises a hydrotrope that is selected from the group consisting of sodium benzoate, sodium toluene sulphonate, sodium cumene sulphonate, sodium xylene sulphonate, sodium salicylate, sodium acetate, and mixtures thereof. The more preferred hydrotropes are sodium benzoate, sodium acetate and sodium salicylate. The hydrotrope is preferably present in 0.2 to 20%, more preferably 0.2 to 8 %, further more preferably 1 to 4 % by weight of the composition.

Without wishing to be bound by theory, it is believed that very low concentration of the essential oil actives can be used since the polymer and the hydrotrope interact synergistically through different mechanisms e.g. the polymer ensuring that there is entrapment of actives thereby increasing local concentration and the hydrotrope enhances the entrapment through formation of a complex with the essential oil actives thereby providing an antibacterial composition that gives the desired action using minimal amount of actives, while ensuring that sensory issues of off odour, irritation are minimized, while ensuring affordability in terms of low cost are provided to the consumer.

The antibacterial composition of the invention may be used to develop various personal care and household care products. Examples include personal care compositions in the form of creams, lotions and gels which may provide various other benefits like moisturization, sunscreening and skin lightening benefits, malodour control and antiperspirancy. The antibacterial benefits are afforded by the composition of the invention in very fast times e.g. in less than 5 minutes, often in less than one minutes, in some cases in less than 30 seconds and in certain other cases in less than 15 seconds. This fast acting antibacterial composition is especially suited for incorporation in wash-off products e.g. soaps in the form of bars, liquids and gels. These products may be used for personal cleansing e.g. as personal wash soap bars, body wash liquids, shower gels, hand wash liquids, gels and lotions, and as face wash products. It is preferred that the composition of the invention is formulated to have a pH of 3 to 11 preferably 3.5 to 8.0 where the efficacy of the synergistic interaction between the antibacterial actives, the polymer and the hydrotrope is seen to be maxiumum. The composition may also be used for providing disinfection benefits other personal substrates like hair and the oral cavity. Thus the composition may be used to formulate shampoos, conditions and mouthwashes.

The composition of the invention may be used for cleaning hard surfaces and thus may be formulated as a floor cleaner, toilet cleaner or a gel or emulsion for cleaning surfaces in the kitchen like table tops, utensils, crockery or as an oven cleaner.

Particularly preferred carriers for formulating the composition of the invention in the various products mentioned above are water or oil/solvent, more preferred carrier being a mixture of water and oil. In most of the envisaged applications like personal care/washing, oral care and hard surface cleaning, the antibacterial composition may be formulated in an aqueous base (water being the carrier) e.g. products in gel format or in purely oil/solvent base e.g. products in anhydrous stick form or propellant containing products. However, most preferred product format has an emulsion base (water and oil being the carriers) e.g. soap products in liquid, solid, lotion or semisolid form for hand wash, face wash, body wash, or shaving applications; toothpaste/ dentifrices for oral care applications or products for hard surface cleaning in bars or liquids form.

The antibacterial composition preferably comprises 1 to 80% surfactant. In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

A particularly preferred surfactant is soap. Soap is a suitable surfactant for personal washing applications of the antibacterial composition of the invention. The soap is preferably C8-C24 soap, more preferably C10-C20 soap and most preferably C12-C16 soap.

The antibacterial composition of the invention is useful in hard surface cleaning applications. In such applications preferred surfactants are nonionic surfactants, such as C8-C22, preferably C8-C16 fatty alcohol ethoxylates, comprising between 1 and 8 ethylene oxide groups when the product is in the liquid form. When the product is in the solid form for hard surface cleaning applications, surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16. Suitable surfactant concentrations in liquid forms of hard surface cleaning application are generally from about from 0.5 to 10%, preferably from 1 to 5 % by weight of the composition. In solid compositions, surfactant is preferably present in 5 to 40%, preferably from 10 to 30% by weight of the composition.

The antibacterial composition of the invention is useful in oral care compositions e.g. in a dentifrice/ toothpaste or oral rinse product. In such applications, preferred surfactants are anionic, nonionic or amphoteric in nature, preferably anionic or amphoteric. Anionic surfactant is preferably an alkali metal alkyl sulphate, more preferably a sodium lauryl sulphate (SLS). Mixtures of anionic surfactants may also be employed. The amphoteric surfactant is preferably a betaine, more preferably an alkylamidopropyl betaine (wherein the alkyl group is a linear C10∼C18 chain), and most preferably is cocoamidopropyl betaine (CAPB). Mixtures of amphoteric surfactants may also be employed. Suitable surfactant concentrations in oral care application are generally from about 2% to about 15%, preferably from about 2.2% to about 10%, more preferably from about 2.5 to about 5% by weight of the total composition.

Thus, in a highly preferred aspect, the antibacterial compositions include soap, alkyl sulphate or linear alkyl benzene sulphonate as the surfactants.

The composition may further comprise various additional ingredients known to a person skilled in the art. Such additional ingredients include but are not limited to: perfumes, pigments, preservative, emollients, sunscreens, emulsifiers, gelling agents, or thickening agents.

According to one aspect water is a preferred carrier. When water is present, it is preferably present in at least 1%, more preferably at least 2%, further more preferably at least 5% by weight of the composition. Water is preferably present in 10 to 99% in most formulations prepared with the composition of the invention. Most compositions have water from 55 to 99% by weight of the composition.

According to another aspect, inorganic particulate material is also a suitable carrier. When inorganic particulate material is the carrier, the antibacterial composition is in a solid form. Preferably the inorganic particulate material is talc. When the inorganic particulate material is talc, the solid antibacterial composition is particularly useful as a talcum powder for application on face or body.

The invention provides for non-therapeutic benefits. Thus, according to yet another aspect of the invention there is provided non-therapeutic use of a composition comprising 0.01 to 10% by weight an essential oil active selected from eugenol, thymol, geraniol, or terpineol; a polymer selected from the group of consisting of polymers of vinyl alcohol, chitosan and mixtures thereof; and a hydrotrope selected from sodium benzoate, sodium toluene sulphonate, sodium cumene sulphonate, sodium xylene sulphonate, sodium salicylate, or sodium acetate for fast reduction in bacterial count. The desired reduction in bacterial count is preferably achieved in less than 5 minutes, more preferably less than 2 minutes, even more preferably less than 1 minute, still more preferably less than 30 seconds and even more preferably within 15 seconds.

Another aspect of the present invention relates to a non-therapeutic method for providing an anti-bacterial effect to a substrate of interest comprising the steps of (a) applying a composition of the first aspect to the substrate, and (b) waiting for at least 15 seconds.

The method preferably comprises the step of wiping or rinsing the composition from the substrate after the waiting step.

The invention will now be illustrated with the help of the following non-limiting examples.

### EXAMPLES

### Examples 1-6: Compositions to demonstrate the synergistic interaction of the ingredients of the invention

Compositions as shown in Table - 1 were prepared and the compositions were tested for antibacterial efficacy in a 15 seconds contact test, using the following protocol:

### Protocol: Contact kill Assay (15 second contact kill)

The test bacteria *E.coli* ATCC 10536 was grown overnight in TSB broth (Difco - 30gpl) at 37°C for 16hrs. 2ml of this was sub-cultured in 40ml of fresh TSB broth and allowed to grow for 4 hours at 37°C. Then the culture was processed by spinning at 4000 rpm for 5 minutes, washed twice and the cells were then collected. The cell density was adjusted at 620 nm to get the final count of 10⁸ cfu/ml (0.8 OD). The test solutions/formulations were prepared and kept for 3 hours for maturation. 9 ml of the test solution was taken in a sample container and 1 ml of processed culture was added to it. After 15 seconds of contact time, 1 ml of the above mixture was immediately neutralized in D/E broth (Difco - 39gpl). Serial dilution was done in D/E broth and plated on TSA (Difco - 40gpl) in duplicates. In case of the control, 1 ml of test culture was added to 9 ml of saline and was serially diluted and plated on TSA. After solidification, the plates were incubated at 37°C for 48 hrs. The residual colonies were counted after 48 hours incubation and efficacy was calculated by comparing with control. The reduction in the amount of bacteria is depicted as log reduction of bacteria.

**Table - 1**

| Ingredient | Example 1 weight% | Example 2 weight% | Example 3 weight% | Example 4 weight% | Example 5 weight% | Example 6 weight% |
|---|---|---|---|---|---|---|
| Thymol | 0.02 | 0.02 | 0.02 | 0.02 | 0.02 | 0.02 |
| Terpineol | 0.05 | 0.05 | 0.05 | 0.05 | 0.05 | 0.05 |
| PVA | - | - | 0.125 | 0.125 | - | - |
| Chitosan | - | - | - | - | 0.125 | 0.125 |
| Sodium benzoate | - | 2.0 | - | 2.0 | - | 2.0 |
| Water | To 100 | To 100 | To 100 | To 100 | To 100 | To 100 |
| Log reduction | No reduction | 0.3 | 5.0 | 7.2 | 3.2 | 7.2 |

| | | | | | | |
|---|---|---|---|---|---|---|
| PVA is poly vinyl alcohol | | | | | | |

The data in Table - 1 indicates that in the compositions as per the invention (Examples 4 and 6), the ingredients interact synergistically as compared to compositions comprising individual ingredients or binary combinations. (Examples 1 to 3 and 5).

### Examples 7 to 11: Examples demonstrating synergy with other types of polymers and hydrotropes

Several more samples were prepared and the log reduction afforded by these samples were measured and the data is summarized in Table - 2.

**Table - 2**

| Ingredient | Example 1 weight% | Example 7 weight% | Example 8 weight% | Example 9 weight% | Example 10 weight% | Example 11 weight% |
|---|---|---|---|---|---|---|
| Thymol | 0.02 | 0.02 | - | 0.02 | 0.02 | 0.02 |
| Terpineol | 0.05 | 0.05 | - | 0.05 | 0.05 | 0.05 |
| PVA | - | - | - | - | - | - |
| Chitosan | - | - | 0.125 | 0.125 | 0.125 | 0.125 |
| Sodium cumene sulphonate | - | - | - | - | - | 2.0 |
| Sodium salicylate | - | 2.0 | - | - | 2.0 | |
| Water | To 100 | To 100 | To 100 | To 100 | To 100 | To 100 |
| Log reduction | No reduction | 4.1 | 0.1 | 4.5 | 7.2 | 7.2 |

The data in Table - 2 indicates that compositions as per the invention (Examples 10 and 11) give vastly improved antibacterial efficacy as compared to compositions outside the invention (Examples 1 and Examples 7 to 9) and the data indicates synergistic interaction for the examples of the invention.

### Examples 12 to 16: Synergistic interaction when only one essential oil active is used

Several more samples were prepared using geraniol as the essential oil active along with the other ingredients of the invention and the log reduction afforded by these samples were measured and the data is summarized in Table - 3.

**Table - 3**

| Ingredient | Example 12 weight% | Example 13 weight% | Example 14 weight% | Example 15 weight% | Example 16 weight% |
|---|---|---|---|---|---|
| Geraniol | 0.08 | - | 0.08 | - | 0.05 |
| PVA | - | - | - | 0.125 | 0.125 |
| Sodium Benzoate | - | 2.0 | 2.0 | - | 2.0 |
| Log reduction | 0.2 | 0.1 | 4.1 | 0.2 | 7.2 |

The data in Table -3 indicates the synergistic interaction between geraniol (an essential oil active) a hydrotrope and a polymer provides enhanced antibacterial efficacy (Example 16) as compared to compositions outside the invention (Examples 12 to 15).

### Examples 17 to 19: Synergistic interaction when eugenol is used as the essential oil active

Several more samples were prepared using eugenol as the essential oil active along with the other ingredients of the invention and the log reduction afforded by these samples were measured and the data is summarized in Table - 4.

**Table - 4**

| Ingredient | Example 17 weight% | Example 18 weight% | Example 19 weight% |
|---|---|---|---|
| Eugenol | 0.25 | 0.25 | 0.25 |
| PVA | - | - | 0.125 |
| Sodium Benzoate | - | 2.0 | 2.0 |
| Log reduction | 0.3 | 2.9 | 7.3 |

The data in Table -4 indicates synergistic interaction between eugenol (an essential oil active in combination) a hydrotrope and a polymer provides enhanced antibacterial efficacy (Example 19) as compared to compositions outside the invention (Examples 17 and 18).

The invention thus provides for a composition that exhibits vastly improved antibacterial efficacy by synergistic interaction of the ingredients and this is achieved using very low concentration of the essential oil actives.

## Claims

1. An antibacterial composition comprising
(a) 0.01 to 10% by weight of an essential oil active selected from the group consisting of eugenol, thymol, geraniol, terpineol, and mixtures thereof;
(b) a polymer selected from the group consisting of chitosan and polymers of vinyl alcohol and mixtures thereof; and
(c) a hydrotrope selected from the group consisting of sodium benzoate, sodium toluene sulphonate, sodium cumene sulphonate, sodium xylene sulphonate, sodium salicylate, sodium acetate, and mixtures thereof.

2. A composition as claimed in any claim 1 comprising 0.001 to 25% of said polymer by weight of the composition.

3. A composition as claimed in claim 1 or 2 wherein said essential oil active is selected from the group consisting of thymol, terpineol, eugenol and mixtures thereof.

4. A composition as claimed in claim 3 wherein said essential oil active comprises thymol and terpineol.

5. A composition as claimed in any one of the preceding claims wherein said hydrotrope is selected from the group consisting of sodium benzoate, sodium acetate and sodium salicylate.

6. A composition as claimed in any one of the preceding claims comprising 0.2 to 20% of said hydrotrope.

7. A composition as claimed in any one of the preceding claims comprising 55 to 99% of water.

8. A composition as claimed in any one of the preceding claims, wherein the pH of the composition is between 3.0 and 11.0.

9. A non-therapeutic method for providing an anti-bacterial effect to a substrate comprising the steps of:
(a) applying a composition according to any one of preceding claims to the substrate, and
(b) waiting for at least 15 seconds.

10. A non-therapeutic method as claimed in claim 9, wherein the composition is wiped or rinsed from the substrate after step 'b'.

11. Non-therapeutic use of a composition comprising 0.01 to 10% by weight of an essential oil active selected from the group consisting of eugenol, thymol, geraniol, terpineol and mixtures thereof; a polymer selected from the group consisting of chitosan and polymers of vinyl alcohol, and mixtures thereof; and a hydrotrope selected from the group consisting of sodium benzoate, sodium toluene sulphonate, sodium cumene sulphonate, sodium xylene sulphonate, sodium salicylate, or sodium acetate and mixtures thereof; for reduction in bacterial count in less than 5 minutes.

## Patentansprüche

1. Antimikrobielle Zusammensetzung umfassend
(a) 0,01 bis 10 Gew.-% eines essentiellen Ölwirkstoffs, ausgewählt aus der Gruppe bestehend aus Eugenol, Thymol, Geraniol, Terpineol und Mischungen davon;
(b) ein Polymer ausgewählt aus der Gruppe bestehend aus Chitosan und Polymeren von Vinylalkohol und Mischungen davon; und
(c) ein Hydrotrop, ausgewählt aus der Gruppe bestehend aus Natriumbenzoat, Natriumtoluolsulfonat, Natriumcumolsulfonat, Natriumxylolsulfonat, Natriumsalicylat, Natriumacetat und Mischungen davon.

2. Zusammensetzung gemäß Anspruch 1, umfassend 0,001 bis 25% des Polymers, bezogen auf das Gewicht der Zusammensetzung.

3. Zusammensetzung gemäß Anspruch 1 oder 2, wobei der essentiellen Ölwirkstoff aus der Gruppe bestehend aus Thymol, Terpineol, Eugenol und Mischungen davon ausgewählt ist.

4. Zusammensetzung gemäß Anspruch 3, wobei der essentiellen Ölwirkstoff Thymol und Terpineol umfasst.

5. Zusammensetzung gemäß irgendeinem der vorhergehenden Ansprüche, wobei das Hydrotrop aus der Gruppe bestehend aus Natriumbenzoat, Natriumacetat und und Natriumsalicylat ausgewählt ist.

6. Zusammensetzung gemäß irgendeinem der vorhergehenden Ansprüche, umfassend 0,2 bis 20% des Hydrotrops.

7. Zusammensetzung gemäß irgendeinem der vorhergehenden Ansprüche, umfassend 55 bis 99% Wasser.

8. Zusammensetzung gemäß irgendeinem der vorhergehenden Ansprüche, wobei der pH-Wert der Zusammensetzung zwischen 3,0 und 11,0 liegt.

9. Nicht therapeutisches Verfahren, einem Substrat eine antimikrobielle Wirkung zu verleihen, umfassend die Schritte von:
(a) Aufbringen einer Zusammensetzung gemäß irgendeinem der vorhergehenden Ansprüche auf das Substrat, und
(b) mindestens 15 Sekunden lang warten.

10. Nicht therapeutisches Verfahren gemäß Anspruch 9, wobei die Zusammensetzung nach dem Schritt "b" von dem Substrat abgewischt oder abgespült wird.

11. Nicht therapeutische Verwendung einer Zusammensetzung umfassend 0,01 bis 10 Gew.-% eines essentiellen Ölwirkstoffs, ausgewählt aus der Gruppe bestehend aus Eugenol, Thymol, Geraniol, Terpineol und Mischungen davon; ein Polymer ausgewählt aus der Gruppe bestehend aus Chitosan und Polymeren von Vinylalkohol und Mischungen davon; und ein Hydrotrop, ausgewählt aus der Gruppe bestehend aus Natriumbenzoat, Natriumtoluolsulfonat, Natriumcumolsulfonat, Natriumxylolsulfonat, Natriumsalicylat, Natriumacetat und Mischungen davon; zur Verringerung der Keimzahl in weniger als 5 Minuten.

## Revendications

1. Composition antibactérienne comprenant
(a) de 0,01 à 10 % en poids d'un actif d'huile essentielle choisi dans le groupe constitué d'eugénol, de thymol, de géraniol, de terpinéol, et de mélanges de ceux-ci ;
(b) un polymère choisi dans le groupe constitué de chitosane et de polymères d'alcool vinylique et de mélanges de ceux-ci ; et
(c) un hydrotrope choisi dans le groupe constitué de benzoate de sodium, de toluènesulfonate de sodium, de cumènesulfonate de sodium, de xylènesulfonate de sodium, de salicylate de sodium, d'acétate de sodium, et de mélanges de ceux-ci.

2. Composition selon la revendication 1 comprenant de 0,001 à 25 % dudit polymère en poids de la composition.

3. Composition selon la revendication 1 ou 2, dans laquelle ledit actif d'huile essentielle est choisi dans le groupe constitué de thymol, de terpinéol, d'eugénol et de mélanges de ceux-ci.

4. Composition selon la revendication 3, dans laquelle ledit actif d'huile essentielle comprend du thymol et du terpinéol.

5. Composition selon l'une quelconque des revendications précédentes, dans laquelle ledit hydrotrope est choisi dans le groupe constitué de benzoate de sodium, d'acétate de sodium et de salicylate de sodium.

6. Composition selon l'une quelconque des revendications précédentes comprenant de 0,2 à 20 % dudit hydrotrope.

7. Composition selon l'une quelconque des revendications précédentes comprenant de 55 à 99 % d'eau.

8. Composition selon l'une quelconque des revendications précédentes, dans laquelle le pH de la composition et de 3,0 à 11,0.

9. Procédé non-thérapeutique pour fournir un effet antibactérien à un substrat comprenant les étapes consistant :
(a) à appliquer une composition selon l'une quelconque des revendications précédentes au substrat, et
(b) à attendre pendant au moins 15 secondes.

10. Procédé non-thérapeutique selon la revendication 9, dans lequel la composition est essuyée ou rincée du substrat après l'étape 'b'.

11. Utilisation non-thérapeutique d'une composition comprenant de 0,01 à 10 % en poids d'un actif d'huile essentielle choisi dans le groupe constitué d'eugénol, de thymol, de géraniol, de terpinéol et de mélanges de ceux-ci ; un polymère choisi dans le groupe constitué de chitosane et de polymères d'alcool vinylique, et de mélanges de ceux-ci ; et un hydrotrope choisi dans le groupe constitué de benzoate de sodium, de toluènesulfonate de sodium, de cumènesulfonate de sodium, de xylène- sulfonate de sodium, de salicylate de sodium, ou d'acétate de sodium et de mélanges de ceux-ci ; pour une réduction du nombre de bactéries en moins de 5 minutes.
